## Europäisches Patentamt

## European Patent Office

## Office européen des brevets

(11) Veröffentlichungsnummer: **0 032 543**
**A1**

(12) **EUROPÄISCHE PATENTANMELDUNG**

(21) Anmeldenummer: 80107411.3

(22) Anmeldetag: 27.11.80

(51) Int. Cl.³: **C 07 C 11/02,** C 07 C 5/27,
B 01 J 21/20

(30) Priorität: 29.10.80 DE 3040698
10.01.80 DE 3000650

(43) Veröffentlichungstag der Anmeldung: 29.07.81
Patentblatt 81/30

(84) Benannte Vertragsstaaten: **FR GB IT NL**

(71) Anmelder: **CHEMISCHE WERKE HÜLS AG,
Postfach 1320, D-4370 Marl 1 (DE)**

(72) Erfinder: **Franz, Gerhard, Dr., Gersthofener Strasse 18,
D-4370 Marl (DE)**
Erfinder: **Heinrich, Friedrich, Dr., Bitterfelder Strasse 7a,
D-4370 Marl (DE)**
Erfinder: **Ratajczak, Hans-Josef, Dr., Gersthofener
Strasse 14, D-4370 Marl (DE)**

(54) **Verfahren zur Isomerisierung von n-Alkenen.**

(57)    Zur katalytischen Skelettisomerisierung von n-Alkenen
zu iso-Alkenen an fluorierten Aluminiumoxiden als Katalysatoren bei Temperaturen von 250 bis 550°C führt man
die Isomerisierung in Gegenwart von 0,5 bis 150 Gewichtsprozent Wasser, bezogen auf das eingesetzte Alken, durch.
Das aus dem Katalysator ausgetragene Fluor dosiert man
kontinuierlich oder diskontinuierlich mit Hilfe einer flüchtigen Fluorverbindung nach. Den teilweise durch Verkokung desaktivierten Katalysator regeneriert man durch
Abbrennen mit einem sauerstoffhaltigen Gas in Gegenwart
von Wasserdampf.

Graphik 1

Graphik 2

1 : gemäß Erfindung mit H₂O
2 : herkömmlich mit H₂ als Inertgas
3 : herkömmlich ohne H₂O und Inertgas

EP 0 032 543 A1

0032543

## Verfahren zur Isomerisierung von n-Alkenen

Die Erfindung betrifft ein Verfahren zur Skelettisomerisierung von n-Alkenen zu iso-Alkenen, das heißt, zur Umwandlung von unverzweigten in verzweigte ungesättigte Kohlenwasserstoffe.

Die Nachfrage nach iso-Alkenen, insbesondere im $C_4$- bis $C_5$-Bereich, ist in den letzten Jahren durch die Erarbeitung neuer Technologien, durch eine veränderte Rohstoffsituation und durch gesetzgeberische Maßnahmen auf dem Kraftstoffsektor erheblich angestiegen. Beispielhaft für diese Entwicklung sind die Methyl-tert.-Butylether-Synthese, die eine einfache Aufarbeitung des $C_4$-Schnittes unter gleichzeitiger Gewinnung einer wertvollen Kraftstoffkomponente darstellt, sowie Verfahren zur Herstellung von Methacrylsäuremethylester, ausgehend von iso-Buten oder tert.-Butanol.

Die Skelettisomerisierung von n- zu iso-Alkenen ist eine bekannte Reaktion. Die Umwandlung wird normalerweise als heterogen katalysierte Gasphasenreaktion im Temperaturbereich zwischen 300 und 600 $^{\circ}$C durchgeführt. Als Katalysatoren werden saure Feststoffe, z. B. durch geeignete Promotoren sauergestellte Aluminiumoxide, eingesetzt. Das Aluminiumoxid wird bevorzugt in der $\eta$ - bzw. $\gamma$ - Modifikation verwandt.

Problematisch ist jedoch die schnelle, starke Verkokung des Katalysators, die zu sehr kurzen Fahrzeiten zwischen den notwendigen häufigen Regenerierungen führt.

Bei der Isomerisierung von n-Butan zu Isobutan, die technisch in größtem Maßstab durchgeführt wird, dotiert man sogar den Katalysator mit Pd oder Pt und setzt dem Kohlenwasserstoffgemisch Wasserstoff zu, um die in Nebenreaktionen entstehenden Olefine schnellstens wieder zu Alkanen zu hydrieren. Denn ohne diese Maßnahme sinkt die Nutzungsdauer des Katalysators von ca. 1/2 Jahr auf wenige Tage, weil die Olefine zu schneller Koksbildung auf dem Katalysator führen.

Aus diesem Grunde haben die bisher bekanntgewordenen Verfahren zur Buten-Isomerisierung noch zu keiner technischen Realisierung geführt.

Beispielsweise wird in der DE-AS 15 18 580 ein Verfahren zur Buten-Isomerisierung beschrieben, bei dem die Umwandlung in Gegenwart eines 0,5 bis 1,5 Gewichtsprozent Fluor enthaltenden Aluminiumoxids bei 250 bis 550 $^\circ$C durchgeführt wird. Nachteilig an der Reaktionsführung ist die kurze Standzeit des Katalysators. Als Folge von Koksabscheidung geht die Aktivität innerhalb weniger Stunden erheblich zurück. Zur Regenerierung muß der Katalysator mit Luft abgebrannt werden.

In der DE-OS 25 34 459 wird ein Verfahren beschrieben, bei dem das Alken mit einem Katalysator in Kontakt gebracht wird, der durch Umsetzung eines aktivierten Aluminiumoxids mit einem Ester der Kieselsäure erhalten wurde. Entsprechend den Ergebnissen sind auch hier periodische Regenerierungen in Zeiträumen von 10 bis 20 Stunden erforderlich.

In der DE-AS 15 18 584 ist als Maßnahme zur Verlängerung der Standzeit eines fluorierten Aluminiumoxid-Katalysators bekannt, daß man die Isomerisierung in Gegenwart von Wasserstoff als Verdünnungsgas durchführt. Die Halbwertszeit des Katalysators, genommen als der Punkt, wo der Prozentgehalt Isobuten, bezogen auf die Gesamtbutene im gasförmigen Produkt, auf die Hälfte seines Gleichgewichtswertes bei 450 $^\circ$C gefallen ist, wird mit 69 Stunden angegeben.

o.Z. 3616/3691

Bei Einsatz inerter Gase wie $H_2$ oder $N_2$ ist aber die Abtrennung der Inerten von den $C_4$-Kohlenwasserstoffen aufwendig, denn sie erfordert in der Regel den Einsatz von Kälte. Zudem sind bei der notwendigen Regenerierung zusätzliche Sicherheitsmaßnahmen erforderlich, um das Auftreten explosibler Gasgemische zu vermeiden.

Aufgabe der vorliegenden Erfindung ist daher ein Verfahren zur Isomerisierung von n- zu iso-Alkenen, das durch verlängerte Katalysatorbetriebszeiten, das heißt der Fahrzeit zwischen zwei Regenerierungen, und durch einfaches Vorgehen bei der Katalysatorregenerierung ausgezeichnet ist.

Die Aufgabe wird erfindungsgemäß entsprechend den Angaben der Patentansprüche gelöst.

Überraschenderweise gelingt dies durch Zumischung von 0,5 bis 150 Gewichtsprozent Wasser, bezogen auf den n-Alken-Einsatz, die entgegen der Erwartung den Katalysator weder völlig desaktivieren, noch ihn irreversibel schädigen. Vielmehr wird eine gleichmäßige Aktivität eingestellt, die auch über sehr lange Zeiträume beibehalten wird und eine hohe Selektivität bei der Isomerisierung zu den Isoalkenen, beispielsweise zum Isobuten, mit sich bringt.

Entgegen allen Erwartungen wird das Fluor durch die großen Wassermengen nicht spontan als HF ausgetragen. Während das Chlor in Reforming-Katalysatoren bereits durch kleine Wassermengen schnell ausgetragen wird, wird das Fluor auch durch große Wassermengen nur in ganz geringem Umfang ausgetragen. In der Wasserphase hinter dem Reaktor werden nur wenige ppm Fluor gefunden. Eine allmähliche Schädigung des Katalysators macht sich erst nach mehreren Betriebstagen bemerkbar.

Völlig vermieden wird diese allmähliche Schädigung, wenn man die aus dem Katalysator ausgetragene geringe Menge Fluor mit Hilfe einer dem Einsatzgemisch zugefügten flüchtigen Fluorverbindung, beispielsweise HF, ersetzt. HF kann man dem Frischwasser in einer dem Fluoraustrag entsprechenden Konzentration zusetzen. Im einfachsten Fall wird das Reaktionswasser, eventuell nach einer Filterung, im Kreis gefahren.

Die Nachfluorierung des Katalysators kann auch durch Zugabe organischer Fluorverbindungen wie z. B. Tetrafluorkohlenstoff oder Fluoroform erfolgen. Diese werden ebenfalls dem Fluoraustrag entsprechend dem Einsatzgemisch zudosiert. Grundsätzlich kann die Nachfluorierung kontinuierlich, entsprechend dem Austrag, oder diskontinuierlich erfolgen.

Das ausgetragene Fluor wird ausschließlich in der Wasserphase wiedergefunden. Die Bildung von Organo-Fluor-Verbindungen wird nicht beobachtet.

Durch die Zugabe von 0,5 bis 150 Gewichtsprozent Wasser zum Einsatzolefin kann die Betriebszeit zwischen zwei Regenerierungen, die in Folge der teilweisen Verkokung des Katalysators erforderlich sind, wesentlich verlängert werden.

Diese Regenerierung des teilweise durch Verkokung desaktivierten Katalysators erfolgt durch Abbrennen mit einem sauerstoffhaltigen Gas in Gegenwart von Wasserdampf. Gegenüber der Fahrweise ohne Wasser bzw. dem Zusatz äquimolarer Stickstoff- oder Wasserstoffmengen kann die Halbwertszeit bei vergleichbarem Anfangsumsatz bis zu einem Faktor 10 und mehr gesteigert werden. Eine Desaktivierung kann durch höheren Wasserzusatz sogar völlig vermieden werden, wenn konstante, allerdings geringere Umsätze interessant erscheinen. Die geeigneten Wassermengen liegen im Bereich von 0,5 bis 150 Gewichtsprozent, bevorzugt von 2 bis 130 Gewichtsprozent, bezogen auf eingesetztes Alken. Wassermengen oberhalb 150 Gewichtsprozent führen zu keiner weiteren Verbesserung bzw. zu technisch nicht mehr interessanten kleinen Umsätzen. Bei Wassermengen unterhalb von 0,5 Gewichtsprozent ist der gewünschte Effekt zu gering.

Neben der Verlängerung der Halbwertszeit bewirkt die Wasserzugabe zusätzlich eine Steigerung der Selektivität. Dies ist auf eine Abnahme an Crack- sowie insbesondere Aromaten und Oligomer-Produkten zurückzuführen. Beide Nebenreaktionen sind sicherlich maßgeblich an der Verkokung des Katalysators beteiligt. Entsprechend ist die Bildung dieser Produkte im direkten Zusammenhang zur Halbwertszeit zu sehen.

Als n-Alkene eignen sich Buten-1, Buten-2, n-Pentene sowie Alkene mit einer Kohlenstoffanzahl von 6 bis 16. Bevorzugt setzt man $C_4$-n-Alkene wie Buten-1 und Buten-2 ein.

Zur Durchführung der vorliegenden Erfindung wird ein Alken oder eine Alkenmischung, möglicherweise auch in Anwesenheit von Alkanen oder anderen inerten Gasen wie Stickstoff oder $CO_2$, mit Wasserdampf homogenisiert und bei Temperaturen von 250 bis 550 °C, vorzugsweise von 350 bis 500 °C über fluorierte Aluminiumoxide geleitet. Die Herstellung der fluorierten Aluminiumoxide erfolgt nach dem Stand der Technik (DE-AS 15 18 580) und ist nicht Gegenstand dieser Anmeldung.

Die Reaktion kann sowohl im Festbett als auch im Wirbelbett durchgeführt werden. Der Reaktionsdruck liegt im allgemeinen im Bereich von atmosphärischem Druck bis 10 bar. Man kann jedoch auch bei Unterdruck arbeiten. Bei Arbeiten unter Druck, bevorzugt bei 3 bis 7 bar, setzt man vorzugsweise 51 bis 150 Gewichtsprozent, insbesondere 55 bis 130 Gewichtsprozent Wasser ein.

Die Raumdurchsatzgeschwindigkeit, ausgedrückt als LHSV (Liquid hourly space velocity), liegt im Bereich von 0,1 bis 20 und vorzugsweise von 0,2 bis 10 $\underline{/}$1/1 . h$\underline{/}$.

Die Regenerierung des teilweise verkokten Katalysators durch Überleiten eines sauerstoffhaltigen Gases, z. B. Luft, erfolgt ebenfalls in Gegenwart von Wasserdampf. Im einfachsten Fall wird nur die Olefindosierung unterbrochen und der Reaktor durch Weiterleiten des Wasserdampfes inertisiert, wobei man den Druck aufrecht erhält. Dann wird das sauerstoffhaltige Gas zugemischt, bis der Katalysator freigebrannt ist. Nach erneuter Inertisierung durch reinen Wasserdampf wird wieder Olefin zudosiert. Es ist zweckmäßig, vor Zudosieren des Olefins - insbesondere bei frisch eingefülltem Katalysator - den Katalysator eine gewisse Zeit nur mit Wasserdampf zu behandeln. Durch dieses Vorgehen wird das Spülen mit einem separaten Inertgas überflüssig und somit die Zahl der notwendigen Schalt- und Regelvorgänge deutlich verringert.

Ein weiterer Vorteil ist die gute Wärmeleitfähigkeit des Wasserdampfes. Dadurch wird die Reaktionswärme des Abbrennvorganges besser abgeführt und somit das Auftreten stark überhitzter Zonen vermieden. Gleichzeitig kann die Abbrenngeschwindigkeit gesteigert werden, was zu kürzeren Regenerationszeiten und somit zu einer besseren Ausnutzung der Anlage führt.

Bei Arbeiten unter Druck müssen größere Wassermengen als bei Normaldruck angewandt werden - beispielsweise 25 bis 150 Gewichtsprozent bei einem Druck von 6 bar.

Bei Einsatz von Gemischen mit viel Alken-1, beispielsweise Buten-(1) oder viel cis-Alken-2, beispielsweise cis-Buten-(2), ist es von Vorteil, der Skelettisomerisierung gemäß vorliegender Erfindung eine Doppelbindungsisomerisierung nach bekannten Verfahren vorzuschalten, um die Wärmeentwicklung im Katalysatorbett zu erniedrigen, um Temperaturspitzen durch ungleichmäßige Wärmeentwicklung im Katalysatorbett zu vermeiden und damit eine annähernd isotherme Reaktionsführung zu ermöglichen.

Es ist ein besonderer Vorteil des Verfahrens, daß man bei der Druckfahrweise, vorzugsweise mit größeren Wassermengen, Gaskompressoren für die Rückkomprimierung (nach der Isomerisierung) einsparen kann, da beispielsweise die $C_4$-Alkene in der Technik unter Druck vorliegen und eingesetzt werden, wie z. B. bei der Herstellung von MTB.

Die erhaltenen iso-Alkene, beispielsweise das iso-Buten, verwendet man zur Herstellung wertvoller Kraftstoffkomponenten sowie zur Herstellung von Methacrylsäuremethylester.

Das erfindungsgemäße Verfahren wird durch die folgenden Beispiele näher erläutert.

Vergleichsbeispiele 1 und 2

In bekannter Weise stellt man einen fluorierten Aluminiumoxidkatalysator her, indem man $\eta$-$Al_2$-$O_3$ mit wäßriger Ammoniumfluoridlösung aufschlämmt, trocknet und bei 400 bis 500 °C calciniert.

Der F-Gehalt des Katalysators beträgt 0,7 %. Zur Durchführung aller folgenden Versuche füllt man 185 ml dieses Katalysators in ein Fixbett (Röhrenreaktor mit einem Innendurchmesser von 3,0 cm). Die Beheizung des Reaktors erfolgt durch ein elektrisch beheiztes Wirbelbad.

Den $C_4$-Kohlenwasserstoff und das evtl. eingesetzte inerte Zusatzgas dosiert man mittels mechanischer Mengenregler. Die Beschickung wird mit leichtem Vordruck von 1,1 bar (absolut) bei 450 °C über das Katalysatorbett geleitet.

0032543

O.Z. 3618/3691

Die Raumdurchsatzgeschwindigkeit, ausgedrückt als LHSV, beträgt 1,3 1/1 . h.

Der Produktstrom wird bei 10 °C durch ein Fallensystem geleitet, um Flüssigprodukt auszukondensieren. Die Zusammensetzung der gasförmigen Produkte wird gaschromatographisch bestimmt. Der Anteil der größer $C_5$-Produkte wird gravimetrisch ermittelt.

Zur Durchführung der Analytik werden folgende Definitionen aufgestellt:

$$\text{Umsatz \%} = \frac{[(BT)\,\text{ein} - (BL)\,\text{aus}] \times 100}{(BT)\,\text{ein}}$$

$$\text{Selektivität für Isobuten \%} = \frac{(\text{Isobuten})\,\text{aus} \times 100}{[(BT)\,\text{ein} - (BL)\,\text{aus}]}$$

$$\text{Ausbeute an Isobuten \%} = \frac{\text{Umsatz \%} \times \text{Selektivität \%}}{100}$$

$$= \frac{(\text{Isobuten})\,\text{aus} \times 100}{(BT)\,\text{ein}}$$

$$\text{Selektivität für } <C_4 + \text{gesättigte KW} = \frac{(<C_4 + \text{gesättigte KW})\,\text{aus} \times 100}{[(BT)\,\text{ein} - (BL)\,\text{aus}]}$$

$$\text{Selektivität für } C_5 + \text{KW} = \frac{(C_5 + \text{KW})\,\text{aus} \times 100}{[(BT)\,\text{ein} - (BL)\,\text{aus}]}$$

Die vorstehenden Symbole haben die folgende Bedeutung:

BL       = Menge lineare Butene
         = trans-Buten + cis-Buten + 1-Buten

BT       = Menge eingesetztes Buten

$< C_4$ + gesättigte Kohlenwasserstoffe (KW)
         = Menge $C_1$ + $C_2$ + $C_3$ + Isobutan + n-Butan

$C_5$ + KW = Menge der KW mit mehr als 4 Kohlenstoffatomen

Die Symbole ein und aus bedeuten jeweils eingeführt in bzw. ausgetragen aus dem Reaktor.

Bei den Vergleichsbeispielen sowie bei den Beispielen 1 bis 3 wird als Edukt 1-Buten mit einer Reinheit > 99,8 % eingesetzt.

Im Vergleichsbeispiel 1 setzt man reines 1-Buten ein. Die Ergebnisse sind in Tabelle 1 wiedergegeben.

Im Vergleichsbeispiel 2 mischt man dem 1-Buten 30 Molprozent (bezogen auf 1-Buten) Wasserstoff zu. Die Ergebnisse sind in Tabelle 2 wiedergegeben.

Beispiel 1

Man setzt den in den Vergleichsbeispielen beschriebenen Katalysator ein (Druck ebenfalls 1,1 bar). Dem 1-Buten setzt man 30 Molprozent, entsprechend 10 Gewichtsprozent (bezogen auf 1-Buten), Wasser zu. Das Wasser wird verdampft und vor dem Reaktor in einem Statik-Mischer mit dem Buten homogenisiert. Das Wasser enthält Fluor in einer Konzentration (eingesetzt als HF), die der Fluor- konzentration in der Wasserphase hinter dem Reaktor entspricht (40 Gewichts-ppm). Die Ergebnisse sind in Tabelle 3 wiedergegeben.

Die Gegenüberstellung des Beispiels 1 mit den Vergleichsbeispielen zeigt, daß bei Wasserzusatz zum 1-Buten die Desaktivierung des Katalysators wesentlich langsamer erfolgt. Gleichzeitig ist die Selektivität gegenüber der Fahrweise ohne Wasser erheblich besser. Besonders deutlich wird der Vorteil des Wasserzusatzes bei der Butenisomerisierung durch die Graphiken 1 bis 3. Dort sind die wesentlichen Kenngrößen der Reaktion (Umsatz, Isobutenselektivität, Isobutenausbeute) als Funktion der Zahl der Betriebsstunden aufgetragen.

Der Katalysator aus diesem Beispiel wird unter den dort angegebenen Bedingungen jeweils 50 Stunden betrieben und dann regeneriert. Die Regenerierung erfolgt durch Abbrennen mit Luft (Luftmenge 15 Nl/h; 2 Stunden) bei der gleichen Badtemperatur unter Beibehaltung der Wasserdosierung (F-Gehalt im $H_2O$ als HF: 40 Gewichts-ppm).

Die Versuchsergebnisse in Tabelle 4 erhält man nach zwölfmaligem Regenerieren und einer Gesamtbetriebsdauer (Summe der Betriebszeiten zwischen den Regenerierungen) von etwa 700 Stunden. Die Ergebnisse zeigen, daß der Katalysator keine Schädigung durch den Wasserzusatz erfährt, wenn Fluor, entsprechend dem Austrag, nachdosiert wird.

Zu gleichen Ergebnissen kommt man, wenn man an Stelle der Fluornachdosierung das Wasser im Kreis fährt.

O.Z. 3616/3691

Vergleichsbeispiel 1: Isomerisierung von 1-Buten
ohne Zusatz von Inerten und $H_2O$

Tabelle 1

| Temperatur $^{\circ}C$ | 450 | 450 | 450 | 450 | 450 | 450 |
|---|---|---|---|---|---|---|
| Betriebsstunden | 1 | 2 | 6 | 8 | 20 | 30 |
| Raumdurchsatzgeschwindigkeit 1/1 h | 1,3 | 1,3 | 1,3 | 1,3 | 1,3 | 1,3 |
| Umsatz % | 54,9 | 46,7 | 31,6 | 26,2 | 10,1 | 4,0 |
| Selektivität (Isobuten) % | 54,9 | 62,2 | 65,5 | 65,9 | 66,9 | 67,4 |
| Ausbeute (Isobuten) % | 30,1 | 29,0 | 20,7 | 17,3 | 6,7 | 2,7 |
| Selektivität ($<C_4$ + ges. KW) % | 9,8 | 7,5 | 6,7 | 6,4 | 5,5 | 5,4 |
| Selektivität ($C_5{}^+$) % | 33,4 | 28,1 | 27,5 | 27,0 | 25,8 | 25,1 |

Vergleichsbeispiel 2: Isomerisierung von 1-Buten unter Zusatz von 30 Molprozent $H_2$, aber ohne $H_2O$

Tabelle 2

| | | | | | | |
|---|---|---|---|---|---|---|
| Temperatur °C | 450 | 450 | 450 | 450 | 450 | 450 |
| Betriebsstunden | 1 | 2 | 6 | 8 | 20 | 30 |
| Raumdurchsatzgeschwindigkeit 1/1 h | 1,3 | 1,3 | 1,3 | 1,3 | 1,3 | 1,3 |
| Umsatz % | 53,8 | 49,7 | 38,6 | 34,2 | 17,1 | 8,3 |
| Selektivität (Isobuten) % | 57,6 | 60,3 | 65,9 | 66,6 | 69,5 | 70,0 |
| Ausbeute (Isobuten) % | 31,0 | 30,0 | 25,4 | 22,8 | 11,9 | 5,8 |
| Selektivität ($<C_4$ + ges. KW) % | 10,1 | 7,4 | 6,2 | 6,0 | 5,1 | 5,0 |
| Selektivität ($C_5{}^+$) % | 30,3 | 30,1 | 25,2 | 25,0 | 23,2 | 22,8 |

0032543

Beispiel 1: Isomerisierung von 1-Buten unter Zusatz von 30 Molprozent entsprechend 10 Gewichtsprozent $H_2O$

Tabelle 3

| Temperatur $^{\circ}C$ | 450 | 450 | 450 | 450 | 450 | 450 |
|---|---|---|---|---|---|---|
| Betriebsstunden | 1 | 2 | 6 | 8 | 20 | 30 |
| Raumdurchsatzgeschwindigkeit 1/1 h | 1,3 | 1,3 | 1,3 | 1,3 | 1,3 | 1,3 |
| Umsatz % | 51,0 | 49,7 | 48,2 | 47,3 | 41,1 | 38,9 |
| Selektivität (Isobuten) % | 63,8 | 64,9 | 68,8 | 70,4 | 74,1 | 75,0 |
| Ausbeute (Isobuten) % | 32,5 | 32,3 | 33,1 | 33,2 | 30,5 | 29,2 |
| Selektivität ($<C_4$ + ges. KW) % | 9,6 | 8,8 | 8,4 | 8,0 | 7,1 | 7,1 |
| Selektivität ($C_5^+$) % | 25,8 | 25,3 | 21,9 | 20,5 | 17,0 | 16,5 |

0032543

Beispiel 1: Isomerisierung von 1-Buten unter Zusatz von 30 Molprozent entsprechend 10 Gewichtsprozent $H_2O$ nach 12 Regenerierungen

Tabelle 4

| | | | | | |
|---|---|---|---|---|---|
| Temperatur $^{o}C$ | 450 | 450 | 450 | 450 | 450 |
| Betriebsstunden | 1 | 2 | 8 | 20 | 32 |
| Raumdurchsatzgeschwindigkeit 1/1 h | 1,3 | 1,3 | 1,3 | 1,3 | 1,3 |
| Umsatz % | 50,4 | 49,4 | 47,0 | 41,2 | 38,6 |
| Selektivität (Isobuten) % | 64,4 | 65,4 | 71,1 | 76,9 | 77,0 |
| Ausbeute (Isobuten) % | 32,5 | 32,3 | 33,4 | 31,7 | 29,7 |
| Selektivität ($<C_4$ + ges. KW) % | 9,8 | 8,7 | 8,0 | 7,0 | 7,0 |
| Selektivität ($C_5{}^{+}$) % | 23,9 | 23,8 | 18,9 | 14,6 | 14,0 |

0032543

O.Z. 3616/3691

0032543

## Beispiel 2

Der in Beispiel 1 bereits eingesetzte Katalysator wird unter den dort angegebenen Bedingungen weiter betrieben, wobei das Reaktionswasser im Kreis gefahren wird. Nach achtmaligem Regenerieren und einer Gesamtbetriebszeit von 400 Stunden beobachtet man keine Schädigung des Katalysators. Man erhält die gleichen Ergebnisse wie in Beispiel 1.

## Beispiel 3

Der in Beispiel 2 bereits eingesetzte Katalysator wird unter den dort angegebenen Bedingungen betrieben, wobei Frischwasser (ohne Fluor) zugefahren und der Fluoraustrag durch Zugabe einer entsprechenden Menge Tetrafluormethan ($44$ ppm $= 44$ mg $CF_4/1$ $H_2O$) kompensiert wird. Zur Dosierung werden jeweils 180 mg $CF_4$ in 40 kg 1-Buten gelöst. Nach achtmaligem Regenerieren und einer Gesamtbetriebszeit von 400 Stunden erhält man die gleichen Ergebnisse wie in Beispiel 1.

## Beispiel 4

Der in Beispiel 3 bereits eingesetzte Katalysator wird unter den in Beispiel 1 angegebenen Bedingungen betrieben, wobei man anstelle von 1-Buten einen $C_4$-Schnitt folgender Zusammensetzung einsetzt:

| | |
|---|---|
| iso-Buten | 7,31 Gewichtsprozent |
| n-Butan | 23,43 Gewichtsprozent |
| 1-Buten | 43,92 Gewichtsprozent |
| trans-Buten-(2) | 15,50 Gewichtsprozent |
| cis-Buten-(2) | 9,80 Gewichtsprozent |

Entsprechend den Versuchsergebnissen verhalten sich die Butane wie Inerte. Die auf eingesetzte Butene bezogenen Umsätze und Selektivitäten (s. Tabelle 5) entsprechen im wesentlichen den Versuchen mit reinem 1-Buten.

Beispiel 4: Isomerisierung eines $C_4$-Schnittes
unter Zusatz von 10 Gewichtsprozent $H_2O$

Tabelle 5

| | | | | | |
|---|---|---|---|---|---|
| Temperatur $^{o}C$ | 450 | 450 | 450 | 450 | 450 |
| Betriebsstunden | 1 | 2 | 8 | 20 | 30 |
| Raumdurchsatzgeschwindigkeit 1/1 h | 1,3 | 1,3 | 1,3 | 1,3 | 1,3 |
| Umsatz % | 49,6 | 48,9 | 46,6 | 38,6 | 35,8 |
| Selektivität (Isobuten) % | 68,7 | 68,7 | 72,9 | 76,8 | 79,8 |
| Ausbeute (Isobuten) % | 34,0 | 33,6 | 33,9 | 29,6 | 28,6 |
| Selektivität ($<C_4$ + ges. KW) % | 9,6 | 9,3 | 8,2 | 7,3 | 7,0 |
| Selektivität ($C_5{}^{+}$) % | 21,1 | 20,2 | 17,9 | 13,8 | 12,9 |

O.Z. 3616/3691

## Beispiel 5

Man setzt den in den Beispielen 1 bis 4 beschriebenen Katalysator ein (Druck 6,0 bar). Dem 1-Buten gibt man 400 Molprozent entsprechend 129 Gewichtsprozent (bezogen auf 1-Buten) Wasser zu. Das Wasser wird verdampft und vor dem Reaktor in einem Statik-Mischer mit dem Buten homogenisiert. Das Wasser enthält Fluor in einer Konzentration (eingesetzt als HF), die der Fluorkonzentration in der Wasserphase hinter dem Reaktor entspricht (90 Gewichts-ppm). Dies erreicht man durch Zudosieren von 0,215 ml HF 40-proz/1 $H_2O$. Die Ergebnisse sind in Tabelle 6 wiedergegeben.

Der Katalysator aus diesem Beispiel wird unter den dort angegebenen Bedingungen 100 Stunden betrieben und dann regeneriert. Die Regenerierung erfolgt durch Abbrennen mit Luft (Luftmenge 15 Nl/h; 2 Stunden) bei der gleichen Badtemperatur unter Beibehaltung der Wasserdosierung (F-Gehalt im $H_2O$ als HF: 90 Gewichts-ppm). Die Wärmetönung bei der Regenerierung war nach Minuten beendet.

Die Versuchsergebnisse in Tabelle 7 erhält man nach zwölfmaligem Regenerieren und einer Gesamtbetriebsdauer (Summe der Betriebszeiten zwischen den Regenerierungen) von etwa 1000 Stunden. Die Ergebnisse zeigen, daß der Katalysator keine Schädigung durch den Wasserzusatz erfährt, wenn Fluor, entsprechend dem Austrag, nachdosiert wird.

Zu gleichen Ergebnissen kommt man, wenn man anstelle der Fluornachdosierung das Wasser im Kreis fährt.

Beispiel 5 : Isomerisierung von 1-Buten bei 6 bar unter Zusatz von 400 Molprozent entsprechend 129 Gewichtsprozent Wasser

Tabelle 6

| | 450 | 450 | 450 | 450 | 450 |
|---|---|---|---|---|---|
| Temperatur $^{\circ}$C | 450 | 450 | 450 | 450 | 450 |
| Betriebsstunden | 1 | 2 | 24 | 45 | 100 |
| Raumdurchsatzgeschwindigkeit 1/1 h | 1,3 | 1,3 | 1,3 | 1,3 | 1,3 |
| Umsatz % | 17,4 | 18,1 | 19,1 | 19,3 | 19,0 |
| Selektivität (Isobuten) % | 94,1 | 93,2 | 94,1 | 93,3 | 93,0 |
| Ausbeute (Isobuten) % | 16,4 | 16,9 | 18,0 | 18,0 | 17,7 |
| Selektivität ($< C_4$ + ges. KW) % | 2,9 | 3,8 | 3,3 | 3,6 | 3,8 |
| Selektivität ($C_5{}^+$) % | 2,9 | 2,7 | 2,5 | 2,6 | 2,5 |

Tabelle 7

| | 450 | 450 | 450 | 450 |
|---|---|---|---|---|
| Temperatur $^{o}$C | 450 | 450 | 450 | 450 |
| Betriebsstunden | 1 | 2 | 20 | 51 |
| Raumdurchsatzgeschwindigkeit 1/1 h | 1,3 | 1,3 | 1,3 | 1,3 |
| Umsatz % | 18,0 | 18,2 | 19,0 | 19,2 |
| Selektivität (Isobuten) % | 93,0 | 93,1 | 93,5 | 93,3 |
| Ausbeute (Isobuten) % | 16,7 | 16,9 | 17,8 | 18,0 |
| Selektivität ($<C_4$ + KW) % | 3,5 | 3,8 | 4,0 | 3,9 |
| Selektivität ($C_5^+$) % | 2,4 | 2,5 | 2,3 | 2,4 |

0032543

Beispiel 6

1-Buten wird zusammen mit 300 Molprozent, entsprechend 96 Gewichtsprozent (bezogen auf 1-Buten) Wasser nach den Angaben des Beispiels 5, jedoch bei 460 °C und einer Raumdurchsatzgeschwindigkeit von 1,1 1/l h umgesetzt. Der Umsatz - bezogen auf eingesetzte Butene - und die Selektivität werden entsprechend Tabelle 8 erhalten.

Die unter 6 bar und bei einer Badtemperatur von 460 °C mit Luft (15 Nl/h) und Fluorid-haltigem Wasserdampf (118 ml $H_2O$ flüssig, F-Gehalt im $H_2O$ als HF: 90 Gewichts-ppm) durchgeführte Regenerierung des Katalysators war nach 120 Minuten (Ende der Wärmetönung) beendet.

Beispiel 6: Isomerisierung von 1-Buten bei 6 bar unter Zusatz von
300 Molprozent entsprechend 96 Gewichtsprozent Wasser

Tabelle 8

| | | | | | | |
|---|---|---|---|---|---|---|
| Temperatur °C | 460 | 460 | 460 | 460 | 460 | 460 |
| Betriebsstunden | 1 | 2 | 17 | 25 | 50 | 100 |
| Raumdurchsatzgeschwindigkeit 1/1 h | 1,1 | 1,1 | 1,1 | 1,1 | 1,1 | 1,1 |
| Umsatz % | 35,1 | 30,0 | 26,5 | 25,7 | 25,0 | 22,9 |
| Selektivität (Isobuten) % | 73,1 | 78,0 | 82,7 | 83,4 | 87,0 | 87,1 |
| Ausbeute (Isobuten) % | 25,7 | 23,4 | 22,0 | 21,4 | 21,8 | 20,0 |
| Selektivität ($< C_4$ + ges. KW) % | 8,5 | 6,8 | 5,4 | 5,1 | 4,8 | 4,9 |
| Selektivität ($C_5^+$) % | 17,7 | 15,1 | 11,3 | 11,0 | 7,7 | 7,3 |

0032543

Beispiel 7

Nach den Angaben des Beispiels 6 werden bei einem Gesamtdruck von 6 bar jedoch bei 470 °C und erhöhter Verweilzeit (Raumdurchsatzgeschwindigkeit 1,1 1/1 h) Buten-1 im Gemisch mit 64 Gewichtsprozent $H_2O$ über den frisch regenerierten Kontakt geleitet. Umsatz (bezogen auf eingesetzte Butene) und Selektivität werden entsprechend Tabelle 9 erhalten. Die Regenerierungszeit des Katalysators (entsprechend Regenerierungsdauer/ Buten-Belastung . Versuchsdauer) beträgt das Zweifache der im Beispiel 6 benötigten.

Beispiel 7: Isomerisierung von 1-Buten bei 6 bar unter Zusatz von 200 Molprozent entsprechend 64 Gewichtsprozent Wasser

Tabelle 9

| | | | | | | |
|---|---|---|---|---|---|---|
| Temperatur $^{\circ}$C | 470 | 470 | 470 | 470 | 470 | 470 |
| Betriebsstunden | 1 | 2 | 17 | 21 | 49 | 100 |
| Raumdurchsatzgeschwindigkeit 1/1 h | 1,1 | 1,1 | 1,1 | 1,1 | 1,1 | 1,1 |
| Umsatz % | 43,1 | 40,1 | 35,5 | 33,8 | 31,0 | 26,1 |
| Selektivität (Isobuten) % | 64,0 | 67,1 | 71,0 | 74,0 | 74,5 | 74,7 |
| Ausbeute (Isobuten) % | 27,6 | 26,9 | 25,2 | 25,0 | 23,1 | 19,5 |
| Selektivität ($<C_4$ + ges. KW) % | 9,4 | 8,1 | 7,3 | 7,5 | 8,5 | 8,2 |
| Selektivität ($C_5^+$) % | 25,7 | 24,8 | 21,0 | 18,5 | 16,8 | 16,3 |

Beispiel 8

Nach den Angaben des Beispiels 6 werden bei einem Gesamtdruck von 6 bar bei 475 °C 1,3 1/1 h eines Gemisches linearer Butene (rückgewonnen aus Isomerisierungsversuchen nach vollständiger Entfernung des Isobutens durch MTB-Bildung; Zusammensetzung 26,5 % 1-Buten, 41,5 % trans-Buten, 31,0 % cis-Buten; geringe Mengen $C_5$- sowie gesättigte $C_4$-KW) im Gemisch mit 200 Molprozent Wasser (64 Gewichtsprozent) am frisch regenerierten Kontakt umgesetzt. Umsatz und Selektivität werden entsprechend Tabelle 10 erhalten. Die Regenerierung des Katalysators nimmt bei gleicher Isomerisierungsdauer sowie vergleichbarer Isobutenausbeute wie im Beispiel 7 eine um den Faktor 0,7 geringere Zeit in Anspruch. Dies sowie die bessere Selektivität sind darauf zurückzuführen, daß bei Verwendung von Butenmischungen statt Buten-1 ein gleichmäßiges Temperaturprofil über den Reaktor und damit eine optimalere Ausnutzung des Kontaktes bei reduzierter "Coke"-Bildung durch Vermeidung von Temperaturspitzen resultiert.

Beispiel 8: Isomerisierung eines n-Buten-Gleichgewichtsgemisches bei 6 bar unter Zusatz von 200 Molprozent entsprechend 64 Gewichtsprozent Wasser

Tabelle 10

| | | | | | |
|---|---|---|---|---|---|
| Temperatur $^{o}C$ | 475 | 475 | 475 | 475 | 475 |
| Betriebsstunden | 1 | 2 | 25 | 50 | 100 |
| Raumdurchsatzgeschwindigkeit 1/1 h | 1,3 | 1,3 | 1,3 | 1,3 | 1,3 |
| Umsatz % | 35,0 | 30,1 | 28,3 | 26,5 | 23,9 |
| Selektivität (Isobuten) % | 70,1 | 72,3 | 77,7 | 79,6 | 82,9 |
| Ausbeute (Isobuten) % | 24,5 | 24,0 | 22,0 | 21,1 | 19,7 |
| Selektivität ($< C_4$ + ges. KW) % | 5,2 | 5,3 | 4,9 | 4,6 | 3,5 |
| Selektivität ($C_5^+$) % | 23,9 | 21,8 | 16,5 | 14,6 | 13,8 |

C0032543

## Beispiel 9

Nach den Angaben des Beispiels 6 wird cis-/trans-Buten (cis-/trans-Verhältnis 27 : 73) mit einer Raumdurchsatzgeschwindigkeit von 1,3 1/1 h bei 6,0 bar und 470 °C im Gemisch mit Wasserdampf (170 Molprozent, entsprechend 55 Gewichtsprozent, bezogen auf Buten-2) am frisch regenerierten Katalysator umgesetzt. Umsatz und Selektivität werden entsprechend Tabelle 11 erhalten. Die unter 6 bar und bei einer Badtemperatur von 470 °C mit 15 Nl/h und gleicher Wasserbelastung wie bei der Isomerisierung durchgeführte Regenerierung war nach 5,5 Stunden beendet.

## Beispiel 10

cis-Buten wird zusammen mit 435 Molprozent, entsprechend 140 Gewichtsprozent Wasser, nach den Angaben des Beispiels 6 bei 480 °C und einer Raumdurchsatzgeschwindigkeit von 1,3 1/1 h umgesetzt. Man erhält konstante Umsätze und Selektivitäten gemäß Tabelle 12. Die unter 6 bar und einer Badtemperatur von 480 °C mit Luft (15 Nl/h) und 215 ml $H_2O$ (Fluorid-haltiger Wasserdampf; F-Gehalt im $H_2O$ als HF: 90 Gewichts-ppm) durchgeführte Regenerierung des Katalysators war nach wenigen Minuten beendet. Dies beweist die praktisch ausbleibende Desaktivierung durch "Coke"-Bildung unter den vorteilhaften Bedingungen hoher Wasserzusätze.

Beispiel 9: Isomerisierung von cis/trans-Buten-2 bei 6 bar unter Zusatz
von 170 Molprozent entsprechend 55 Gewichtsprozent Wasser

Tabelle 11

| | | | | | | |
|---|---|---|---|---|---|---|
| Temperatur $^{\circ}C$ | 470 | 470 | 470 | 470 | 470 | 470 |
| Betriebsstunden | 1 | 2 | 25 | 40 | 58 | 100 |
| Raumdurchsatzgeschwindigkeit 1/1 h | 1,3 | 1,3 | 1,3 | 1,3 | 1,3 | 1,3 |
| Umsatz % | 37,5 | 35,2 | 27,1 | 25,2 | 23,9 | 20,9 |
| Selektivität (Isobuten) % | 65,3 | 68,5 | 73,5 | 74,6 | 76,4 | 82,7 |
| Ausbeute (Isobuten) % | 24,5 | 24,1 | 19,9 | 19,8 | 18,3 | 17,3 |
| Selektivität ($< C_4$ + ges. KW) % | 6,6 | 5,8 | 2,9 | 2,6 | 2,8 | 2,5 |
| Selektivität ($C_5^+$) % | 27,4 | 25,3 | 23,2 | 22,4 | 20,4 | 14,2 |

0032543

O.Z. 3616/3691

Beispiel 10: Isomerisierung von cis-Buten-2 bei 6,0 bar unter Zusatz von 435 Molprozent entsprechend 140 Gewichtsprozent Wasser

Tabelle 12

| | | | | | |
|---|---|---|---|---|---|
| Temperatur °C | 480 | 480 | 480 | 480 | 480 |
| Betriebsstunden | 1 | 2 | 15 | 30 | 50 |
| Raumdurchsatzgeschwindigkeit 1/1 h | 1,3 | 1,3 | 1,3 | 1,3 | 1,3 |
| Umsatz % | 19,1 | 18,9 | 18,4 | 18,1 | 18,0 |
| Selektivität (Isobuten) % | 89,2 | 90,3 | 90,5 | 91,2 | 91,0 |
| Ausbeute (Isobuten) % | 17,0 | 17,1 | 16,7 | 16,5 | 16,4 |
| Selektivität ($< C_4$ + ges. KW) % | 2,0 | 1,8 | 1,4 | 0,8 | 1,2 |
| Selektivität ($C_5^+$) % | 7,9 | 7,5 | 7,3 | 7,6 | 6,9 |

## Beispiel 11

Man setzt an dem im Beispiel 1 eingesetzten Katalysator (185 ml in Röhrenreaktor von 3,0 cm Durchmesser; beheizt mit elektrischem Wirbelbad) bei einem Druck von 1,1 bar und einer Temperatur von 440 $^\circ$C 1,1 l/l h Penten-1 (>99 %) im Gemisch mit Wasserdampf (387 g/l h, entsprechend 55 Gewichtsprozent, bezogen auf Penten) um. Das Wasser enthält Fluor in einer Konzentration (eingesetzt als HF) von 90 Gewichts-ppm. Der Produktstrom wird bei 15 $^\circ$C durch ein Fallensystem geleitet, um Flüssigprodukt auszukondensieren. Die Zusammensetzung der gasförmigen und der $C_5$-Produkte wird gaschromatographisch bestimmt. Der Anteil der größer $C_5$-Produkte wird nach Abdestillieren der $C_5$-Anteile gravimetrisch ermittelt. Zur Durchführung der Analytik werden folgende Definitionen aufgestellt:

$$\text{Umsatz \%} = \frac{\left[(\textstyle\sum PL)\,ein - (\textstyle\sum PL)\,aus\right] \times 100}{(\textstyle\sum PL)\,ein}$$

$$\text{Selektivität für Isopentene \%} = \frac{(\textstyle\sum IP)\,aus \times 100}{\left[(\textstyle\sum PL)\,ein - (\textstyle\sum PL)\,aus\right]}$$

$$\text{Ausbeute an Isopentenen \%} = \frac{\text{Umsatz \% × Selektivität \%}}{100}$$

$$= \frac{(\textstyle\sum IP)\,aus \times 100}{(\textstyle\sum PL)\,ein}$$

$$\text{Selektivität für } {<}C_5 + \text{gesättigte KW} = \frac{({<}C_5 + \text{gesättigte KW})\,aus \times 100}{\left[(PL)\,ein - (PL)\,aus\right]}$$

$$\text{Selektivität für } C_6 + - KW = \frac{(C_6 + KW)\,aus \times 100}{\left[(PL)\,ein - (PL)\,aus\right]}$$

Die vorstehenden Symbole haben folgende Bedeutung:

$\sum PL$ = Summe der linearen Pentene

= Penten-1 + cis-Penten-2 + trans-Penten-2

$\sum IP$ = Summe der verzweigten Pentene

= 2-Methyl-buten-1 + 2-Methyl-buten-2

+ 3-Methyl-buten-1

$<C_5$ + gesättigte Kohlenwasserstoffe (KW)

= Menge $C_1 + C_2 + C_3 + C_4$ + Isopentan + n-Pentan

$C_6$ + KW = Menge der KW mit mehr als 5 Kohlenstoffatomen

Die Symbole "ein" und "aus" bedeuten eingeführt in bzw. ausgetragen aus dem Reaktor.

Das Produkt, das gemäß Tabelle 13 erhalten wird, enthält neben linearen Pentenen, Isopentene sowie einen geringen Anteil an Crack-, gesättigten und $C_6$+-Produkten. Die Verwendung großer Mengen Wasser ermöglicht eine hohe Selektivität an Isoamylenen, die wichtige Chemierohstoffe, z. B. zur Herstellung von Amylphenolen, Oktanzahlverbessernden Ethern und Isoamylalkohol, darstellen.

Beispiel 11: Isomerisierung von Penten-1 bei 1,1 bar unter Zusatz
von 214 Molprozent entsprechend 55 Gewichtsprozent Wasser

Tabelle 13

| | | | | | |
|---|---|---|---|---|---|
| Temperatur °C | 440 | 440 | 440 | 440 | 440 |
| Betriebsstunden | 1 | 2 | 8 | 20 | 30 |
| Raumdurchsatzgeschwindigkeit 1/1 h | 1,1 | 1,1 | 1,1 | 1,1 | 1,1 |
| Umsatz % | 30,4 | 29,9 | 28,7 | 27,8 | 27,2 |
| Selektivität (Isopentene) % | 88,2 | 88,7 | 89,8 | 90,5 | 91,0 |
| Ausbeute (Isopentene) % | 26,8 | 26,5 | 25,8 | 25,2 | 24,7 |
| Selektivität ($< C_5$ + ges. KW) % | 4,2 | 4,0 | 3,5 | 3,4 | 2,8 |
| Selektivität ($C_6$+) % | 8,0 | 7,8 | 7,1 | 6,5 | 6,3 |

C032543

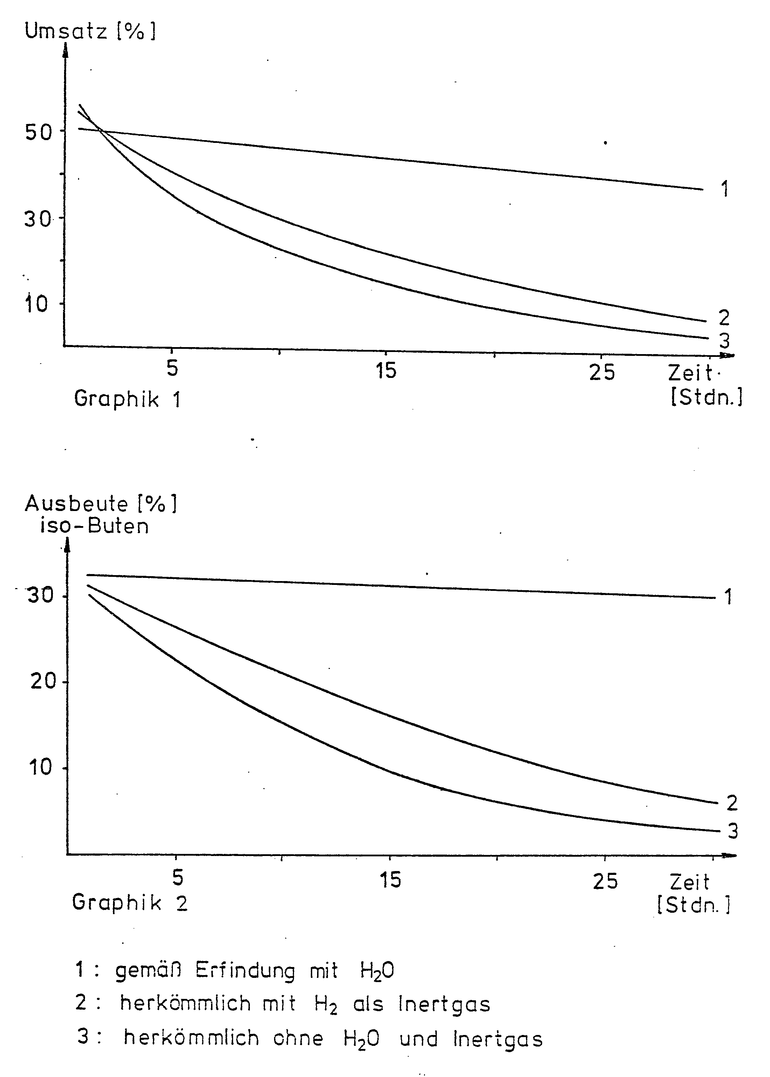

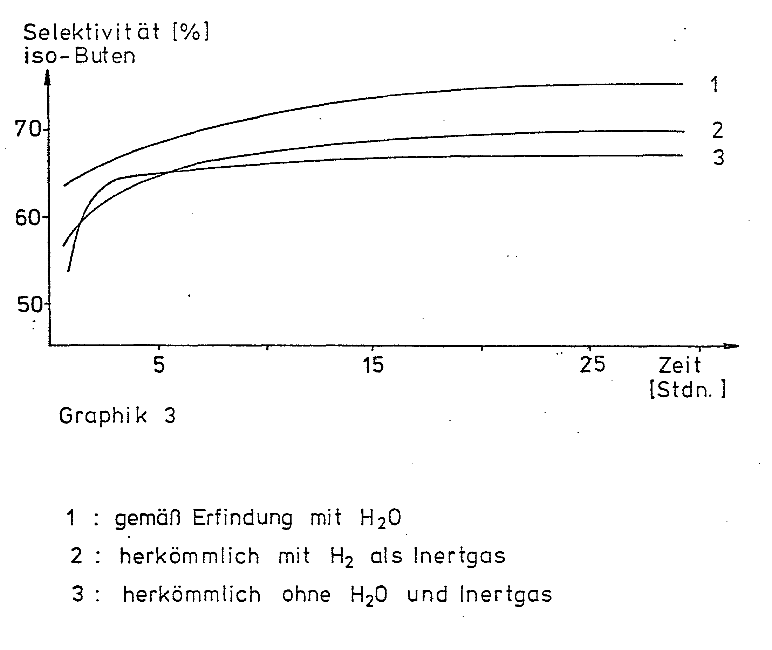

Patentansprüche:

1. Verfahren zur katalytischen Skelettisomerisierung von n-Alkenen zu iso-Alkenen an fluorierten Aluminiumoxiden als Katalysatoren bei Temperaturen von 250 bis 550 °C,
dadurch gekennzeichnet,
daß man die Isomerisierung in Gegenwart von 0,5 bis 150 Gewichtsprozent Wasser, bezogen auf das eingesetzte Alken, durchführt, das aus dem Katalysator ausgetragene Fluor kontinuierlich oder diskontinuierlich mit Hilfe einer flüchtigen Fluorverbindung nachdosiert und den teilweise durch Verkokung desaktivierten Katalysator durch Abbrennen mit einem sauerstoffhaltigen Gas in Gegenwart von Wasserdampf regeneriert.

2. Verfahren nach Anspruch 1,
dadurch gekennzeichnet,
daß man den Fluoraustrag durch eine entsprechende HF-Konzentration im Frischwasser kompensiert.

3. Verfahren nach den Ansprüchen 1 und 2,
dadurch gekennzeichnet,
daß man das Reaktionswasser im Kreis fährt.

4. Verfahren nach den Ansprüchen 1 bis 3,
dadurch gekennzeichnet,
daß das Alken ein Buten, rein oder in Mischung mit anderen Kohlenwasserstoffkomponenten, ist.

5. Verfahren nach den Ansprüchen 1 bis 4,
dadurch gekennzeichnet,
daß man das Alken bei 1 bis 10 bar, vorzugsweise bei 3 bis 7 bar, isomerisiert und den Katalysator unter Druck regeneriert.

O.Z. 3616/3691

0032543

6. Verfahren nach den Ansprüchen 1 bis 5,
   dadurch gekennzeichnet,
   daß man die Isomerisierung in Gegenwart von 2 bis
   130 Gewichtsprozent Wasser durchführt.

Umsatz [%]

0032543

50

30

10

5   15   25   Zeit
[Stdn.]

1

2

3

Graphik 1

Ausbeute [%]
iso-Buten

30

20

10

5   15   25   Zeit
[Stdn.]

1

2

3

Graphik 2

1 : gemäß Erfindung mit $H_2O$

2 : herkömmlich mit $H_2$ als Inertgas

3 : herkömmlich ohne $H_2O$ und Inertgas

O.Z. 3616/3591

0032543

Selektivität [%]
iso-Buten

Graphik 3

1 : gemäß Erfindung mit $H_2O$

2 : herkömmlich mit $H_2$ als Inertgas

3 : herkömmlich ohne $H_2O$ und Inertgas

O.Z. 3616/3691

Europäisches
Patentamt

**EUROPÄISCHER RECHERCHENBERICHT**

0032543

Nummer der Anmeldung

EP 80 10 7411

| | EINSCHLÄGIGE DOKUMENTE | | KLASSIFIKATION DER ANMELDUNG (Int Cl.) |
|---|---|---|---|
| Kategorie | Kennzeichnung des Dokuments mit Angabe, soweit erforderlich, der maßgeblichen Teile | betrifft Anspruch | |
| | US - A - 3 558 733 (J.W. MYERS) <br> * Spalte 2, Zeilen 48-53; Ansprüche * <br><br> -- | 1,4,5 | C 07 C 11/02 <br> 5/27 <br> B 01 J 21/20 |
| | US - A - 3 558 734 (J.W. MYERS) <br> * Spalte 1; Ansprüche * <br><br> -- | 1,4,5 | |
| D | DE - A - 1 518 580 (B.P.) <br> * Ansprüche * <br><br> -- | 1,4 | |
| | DE - A - 2 424 852 (I.C.I.) <br> * Seite 3; Ansprüche * <br><br> -- | 1 | **RECHERCHIERTE SACHGEBIETE (Int. Cl.)** <br><br> C 07 C 5/27 <br> 5/22 <br> B 01 J 21/20 |
| A | US - A - 2 568 964 (C.W. MONT-GOMERY et al.) <br> * Spalte 3; Spalte 5, Zeilen 25-30; Ansprüche * <br><br> ---- | | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 02-04-1981 | VAN GEYT |

EPA form 1503.1 06.78